# EUROPEAN PATENT APPLICATION

(11) **EP 4 101 499 A1**
(43) Date of publication of application: **14.12.2022**
(21) Application number: 22177001.9
(22) Date of filing: 02.06.2022
(51) Int. Cl.: A61N 1/36, A61N 1/375, A61N 1/05

(54) **COCHLEAR HEARING AID IMPLANT INCLUDING AN IMPROVED CONNECTION BETWEEN AN ELECTRODE LEAD AND AN IMPLANT**

(30) Priority: 09.06.2021 EP 21178442
(71) Applicant: Oticon Medical A/S, 2765 Smørum (DK)
(72) Inventor: MARRET, Théo, DK-2765 Smørum (DK)
(74) Representative: Demant

(57) **Abstract**

A cochlear hearing aid implant is disclosed. The cochlear hearing aid implant is configured to stimulate auditory nerves of a recipient of the cochlear hearing aid implant, and the implant includes a transceiver coil configured to at least receive an inductive signal, an electronic circuit configured to provide a stimulation signal based on the inductive signal, an electrode lead including a plurality of electrodes and a first connector, and where the electrode lead is configured to receive the stimulation signal via the first connector and provide the stimulation signal via an electrode of the plurality of electrodes to the auditory nerves. Furthermore, the implant includes a housing configured to accommodate at least the electronic circuit, and wherein the housing includes or is connected to a second connector. The first connector and the second connector are configured to be connected, and wherein the first connector or the second connector includes multiple male plugs arranged in parallel and along a first axis, and wherein the first connector or the second connector includes multiple female plugs arranged in parallel and along a second axis, and where each of the multiple female plugs is configured to be connected to each of the multiple male plug such that the first axis is parallel to the second axis, and where each of the multiple female plugs and each of the multiple male plugs are connected to either the electronic circuit or to an electrode of the plurality of electrodes.

## Description

### FIELD

The present disclosure relates to a cochlear hearing aid implant. More particularly, the disclosure relates to such implant with a connector interface that allows an easy way of connecting and disconnecting an electrode lead to a housing of the cochlear hearing aid implant.

### BACKGROUND

Cochlear hearing aid implants (CIs) are devices containing electrodes inserted in an inner ear (the cochlea) of a patient to recover the sensation of audition to people suffering from severe to profound hearing loss. CIs are bypassing most of the functional hearing chain, and generate series of electrical pulses inside the cochlea to initiate action potentials from the remaining hair cells / spiral ganglions / nerve fibers. Those devices are thus mostly considered as biocompatible electronic machines. Depending on their implementation, they can either be totally implanted or composed of two main parts. A first part is the sound processor, often placed near the ear. It contains microphones that capture the environmental sound, which is processed in real time into a series of codes usable by a second part, implanted into the patient. The implant receives both power and sound information through radiofrequency from the sound processor, and generates electrical pulses sent into the cochlea via electrodes inside the cochlea.

The second part of the cochlear hearing aid implant, is currently made of at least two units: a receiver, configured to accommodate all the electronics and processing of information received from an antenna of the second part; and an electrode array, which sends electrical impulses to the cochlea of the patient. These two units are assembled with each other during manufacturing in a non-reversible way.

When the implant starts failing, both the receiver and the electrode array must be explanted and a new one must be implanted. In a very large proportion of implant failure is related to housing failure, and in that case will result in a removal of the complete cochlear hearing aid implant as it is not possible to disconnect the housing and the electrode array with the known implants. For a patient being born deaf, it is reasonable to claim that he probably will undergo at least 3 revision surgeries. For older patients, there is very often at least one replacement, sometimes two. Each of these surgery will have an impact on the hearing outcomes of the patient. Removing the electrode array from the cochlea and replacing it with a new one is an operation that is very traumatic for the cochlea. As these manipulations damage the cochlea, the hearing performances of the patient drop at each surgery.

As well, current revision surgeries are quite invasive, require significant time (during which infection can occur) and must very often be performed under general anaesthesia (which can have deleterious consequences for the patient, especially if performed many times).

Thus, the possibility to replace the receiver while leaving the electrode array within the cochlea of the cochlea is a crucial stake.

In other medical fields, the technology of being able to disengage components and explant one component instead of all components is known. Some cardiac devices as well as Spinal Cord Stimulation (SCS) devices or Deep Brain Stimulation (DBS) are already equipped with this technology. Just like Cochlear Implants, these devices are intended for long-term use (up to 10 years) and the implantable connector brings terrific benefits to the patient.

However, the case of cochlear implants is more complex for two reasons.

First, the number of channel required for stimulation is much higher for a cochlear implant. DBS and cardiac implants usually are equipped with no more than 4 electrodes. SCS devices usually have between 4 and 8 electrodes, but recent models also have a 2x8 configuration, thus including 16 channels.

Second, the room available for the system is much more limited with a cochlear implant. For cardiac implants and SCS implants, the room for batteries and connectors is much larger. For DBS, the room available on the top of the skull is limited but the IPG (Impulse Generator) can be deported at the top of the back for more space. For cochlear implants, the room available is very limited, including the thin room between the skull and the skin and the room within the mastoidectomy, that is getting smaller as surgical procedures evolve.

For cochlear implants, the number of contacts is very high (ranging from 12 to 24 across CI manufacturers) and there is only very limited room for large batteries (usually worn outside, on the BTE) or connectors. On top of that, the possibility of deportation of the implantable part of the device is limited due to the usability of the external part (which is worn on the ear).

These reasons explain the difficulty to introduce an IC in cochlear implants.

Therefore, there is a need to provide a solution that addresses at least some of the above-mentioned problems. In particular, there is a need to provide a solution that allows a connector interface between the receiver and the electrode array that is not bulky and can be arranged between the skull and the skin of a patient without increasing the complexity of the invasive surgery.

### SUMMARY

According to an aspect of the present disclosure, a cochlear hearing aid implant is disclosed. The cochlear hearing aid implant is configured to stimulate auditory nerves of a recipient of the cochlear hearing aid implant, and the implant includes a transceiver coil configured to at least receive an inductive signal, an electronic circuit configured to provide a stimulation signal based on the inductive signal, an electrode lead including a plurality of electrodes and a first connector, and where the electrode lead is configured to receive the stimulation signal via the first connector and provide the stimulation signal via an electrode of the plurality of electrodes to the auditory nerves. Furthermore, the implant includes a housing configured to accommodate at least the electronic circuit, and wherein the housing includes or is connected to a second connector. The first connector and the second connector are configured to be connected, and wherein the first connector or the second connector includes multiple male plugs arranged in parallel and along a first axis, and wherein the first connector or the second connector includes multiple female plugs arranged in parallel and along a second axis, and where each of the multiple female plugs is configured to be connected to each of the multiple male plug such that the first axis is parallel to the second axis, and where each of the multiple female plugs and each of the multiple male plugs are connected to either the electronic circuit or to an electrode of the plurality of electrodes.

The cochlear hearing aid implant may include at least two multiple male plugs arranged in parallel and along the first axis, and at least two multiple female plugs arranged in parallel and along the second axis.

By arranging multiple female and male plugs in parallel results in a connection interface that is broader than if they were arranged along a single row. However, the length of the single row arrangement of the female plugs and the male plugs will be higher than the width of the parallel plugs, and thereby, the parallel plugs results in a more appropriate design to the cochlear hearing aid implant.

The housing may include a part made of a titanium alloy, such as zirconia, that may comprise the electronic circuit and/or the transceiver coil. Furthermore, the housing may include another part made of a first flexible material that includes at least two fixation means configured to fixate the housing to the skull via at least two screws. The part that includes the titanium alloy may be covered fully or partially by a second flexible material, where the first flexible material and the second flexible material may be made of the same or different material. The flexible material may be silicone. The thickness of the first flexible material may be thinner than the thickness of the second flexible material for the purpose of making the another part more flexible than the part including the electronic circuit.

The second connector may be arranged between the at least two fixation means or connected to the housing between the at least two fixation means. The width of the another part of the housing may not be larger than the width of the part of the housing that includes the electronic circuit and the transceiver coil. The width of the housing is parallel to a transverse axis of the housing and orthogonal to a longitudinal axis of the housing extending through the part of the housing including the electronic circuit and the another part including the fixation means. The at least two fixation means may be partially covered by the first flexible material and connected by the first flexible material. Between the at least two fixation means the second connector may be arranged and within the first flexible material. In another example, the second connector may be connected to the housing and the electronic circuit via a flexible mean that is arranged in the another part and between the two fixation means. The flexible mean may be the another part or extending within the another part guiding the connection of the second connector to the electronic circuit. The connection may be wired.

By arranging the second connector within the another part or the connection of the second connector to the housing between the at least two fixation means, the maximum thickness and the maximum width of the housing along the transverse axis does not increase. In the example where the female and male plugs are arranged along a single row, i.e. a single row plug, the plug extends eventually within the part of the housing including the electronic circuit and the another part resulting in an increase of the maximum width, length and/or thickness of the implant.

The connector interface may include the first connector and the second connector.

The plurality of electrodes includes at least 10 electrodes, and wherein the first connector and the second connector are configured to connect the at least 10 electrodes to the electronic circuit when the first connector and the second connector are connected. The second connector may be connected to the electronic circuit by at least 10 conductive paths, and where each of the at least 10 conductive paths may be configured to receive the stimulation signal and provide it to the electrode lead when the first connector and the second connector are connected. The conductive paths may be partially or fully arranged within the housing of the implant. In a situation where the conductive paths are partially within the housing, an external part of the conductive paths in relation to the housing may be covered by a flexible material, such as silicone. The conductive paths may be applied onto or within a spring mean to avoid any unwanted bending of the conductive paths that may results in damaging the conductive paths.

The at least 10 conductive paths may be arranged within a flexible mean that is connected to the second connector and to the housing. The flexible mean may include the spring mean for similar purpose. By having a flexible mean outside the housing provides the possibility to easily adapt existing cochlear hearing aid implants to the connector interface that includes the first and the second connector.

The housing may have a first thickness, the first connector may have a second thickness and the second connector may have a third thickness, and wherein the first, second and third thickness are determined along a transverse axis being orthogonal to the skull of the patient when the implant is arranged on the skull, and where the second and third thickness are less than the first thickness.

Each of the multiple female plugs may be a hollow tube that includes multiple electrode female contacts distributed along a longitudinal axis of the hollow tube, and when the hollow tube receives one of the multiple male plugs, each of the multiple electrode female contacts connects to one of the plurality of electrodes. In an example where the plurality of electrodes includes at least 10 electrodes, the multiple female plugs includes at least two female plugs where each of the female plugs includes at least 5 electrode female contacts distributed along the longitudinal axis. In an example where the plurality of electrodes includes at least 22 electrodes, the multiple female plugs include at least two female plugs where each of the female plugs includes at least 11 electrode female contacts distributed along the longitudinal axis. In an example where the plurality of electrodes includes at least 23 electrodes, the multiple female plugs include at least two female plugs where a first female plug includes at least 11 electrode female contacts and a second female plug includes at least 12 electrode female contacts distributed along the longitudinal axis. The multiple female plugs include the first and the second female plugs.

Each of the multiple male plugs may include multiple electrode male contacts arranged along a longitudinal axis of each of the multiple male plugs, and wherein each of the multiple male plugs is connected to each of the plurality of electrodes. In an example where the plurality of electrodes includes at least 10 electrodes, the multiple male plugs include at least two male plugs where each of the male plugs includes at least 5 electrode male contacts distributed along the longitudinal axis. In an example where the plurality of electrodes includes at least 22 electrodes, the multiple male plugs includes at least two male plugs where each of the male plugs includes at least 11 electrode male contacts distributed along the longitudinal axis. In an example where the plurality of electrodes includes at least 23 electrodes, the multiple male plugs include at least two male plugs where a first male plug includes at least 11 electrode male contacts and a second male plug includes at least 12 electrode male contacts distributed along the longitudinal axis. The multiple male plugs include the first and the second male plugs.

Each of the multiple electrode female contacts includes a conductive casing with a hollow section configured to deform when receiving one of the multiple male contacts, and wherein the conductive casing is configured to be connected to either the electronic circuit or to an electrode of the plurality of electrodes.

Each of the multiple electrode female contacts may include a conductive casing configured to be connected to either the electronic circuit or to an electrode of the plurality of electrodes, and a flexible member arranged between the conductive casing and an electrode male plug of the multiple male plugs, and wherein the flexible member is configured to deform when receiving one of the multiple male plugs, and wherein the flexible member is made of a conductive material that includes one or more of following material copper, silver-plated copper alloy, stainless steel, Beryllium copper and zirconia copper, gold, silver, nickel or tin.

The conductive casing is made of a conductive material that may be flexible, and the conductive material may include one or more of following material zirconia, copper, silver-plated copper alloy, stainless steel, Beryllium copper and Zirconium copper, gold, silver, nickel or tin. The flexible conductive casing secures an optimal connection between the multiple female contacts and the multiple male contacts. The conductive casing may be shaped as a spring configured to adapt the shape of the hollow section of the conductive casing to the received male contacts of the male plug.

Each of the multiple electrode female contacts connects to each of the multiple electrode male contacts when the hollow tube receives one of the multiple male plugs.

To avoid any unwanted interconnection between the female contacts of the multiple female contacts or between the male contacts of the multiple male contacts an isolator is arranged between the multiple electrode female contacts and/or the multiple electrode male contacts. The multiple female plugs may be applied together via a silicon material acting as an isolator between each of the multiple female plugs.

For preventing any unwanted disconnections between the first and the second connector, the first connector and the second connector may include a locking interface configured to prevent any unwanted disconnections of the first connector and the second connector when being connected. The locking interface may include at least one groove formed into an outer surface of either the first or the second connector and at least one protrusion is arranged on an outer surface of the first or the second connector not having the at least one groove, and wherein the at least one grove is configured to receive the at least one protrusion when connecting the first and the second connector together. The at least one protrusion may be disengaged from the at least one groove when applying a force onto the outer surface of the first and/or the second connector, and wherein the force is applied particularly parallel to the first and second axis. The force may be applied by pressing by hands/fingers onto the outer surface of the first and/or the second connector.

The locking interface may include a hole formed into the first and the second connector, and wherein the locking interface may include a pin connector configured to be inserted into the hole of the first and the second connector when being connected, and wherein the pin connector may be configured to be in a lock position and/or in a disengage position. The pin connector is configured to be in either the lock position or the disengage position when turning the pin connector. The pin connector may include one or more protrusions that is configured to engage with a groove of the first and/or the second connector when turning the pin connector in a direction around a center of the hole in the first and the second connector.

### BRIEF DESCRIPTION OF DRAWINGS

Aspects of the disclosure may be best understood from the following detailed description taken in conjunction with the accompanying figures. The figures are schematic and simplified for clarity, and they just show details to improve the understanding of the claims, while other details are left out. Throughout, the same reference numerals are used for identical or corresponding parts. The individual features of each aspect may each be combined with any or all features of the other aspects. These and other aspects, features and/or technical effect will be apparent from and elucidated with reference to the illustrations described hereinafter in which:
FIGS 1A and 1B illustrate a cochlear hearing aid implant;
FIGS. 2A and 2B illustrate an example of a connection between a housing and an electrode lead;
FIG. 3 illustrates an example of a connection between a first connector and a second connector;
FIGS. 4A to 4F illustrate different examples of a cochlear hearing aid implant;
FIGS. 5A to 5D illustrate different examples of a cochlear hearing aid implant;
FIGS. 6 illustrates an example of a thickness of a housing, a first connector and a second connector;
FIGS. 7A to 7C illustrate an example of a female plug;
FIG. 8 illustrates an example of multiple electrode male plugs;
FIG. 9 illustrates an example of an electrode male plug;
FIG. 10 illustrates an example of multiple electrode female plugs;
FIGS. 11A to 11C illustrate an example of a locking interface; and
FIG. 12 illustrates an example of a locking interface.

### DETAILED DESCRIPTION

The detailed description set forth below in connection with the appended drawings is intended as a description of various configurations. The detailed description includes specific details for the purpose of providing a thorough understanding of various concepts. However, it will be apparent to those skilled in the art that these concepts may be practised without these specific details. Several aspects of the apparatus and methods are described by various blocks, functional units, modules, components, circuits, steps, processes, algorithms, etc. (collectively referred to as "elements"). Depending upon particular application, design constraints or other reasons, these elements may be implemented using electronic hardware, computer program, or any combination thereof.

The electronic hardware may include microprocessors, microcontrollers, digital signal processors (DSPs), field programmable gate arrays (FPGAs), programmable logic devices (PLDs), gated logic, discrete hardware circuits, and other suitable hardware configured to perform the various functionality described throughout this disclosure. Computer program shall be construed broadly to mean instructions, instruction sets, code, code segments, program code, programs, subprograms, software modules, applications, software applications, software packages, routines, subroutines, objects, executables, threads of execution, procedures, functions, etc., whether referred to as software, firmware, middleware, microcode, hardware description language, or otherwise.

A hearing device may include a hearing aid that is adapted to improve or augment the hearing capability of a user by receiving an acoustic signal from a user's surroundings, generating a corresponding audio signal, possibly modifying the audio signal and providing the possibly modified audio signal as an audible signal to at least one of the user's ears. The "hearing device" may further refer to a device such as an earphone or a headset adapted to receive an audio signal electronically, possibly modifying the audio signal and providing the possibly modified audio signals as an audible signal to at least one of the user's ears. Such audible signals may be provided in the form of an acoustic signal radiated into the user's outer ear, or an acoustic signal transferred as mechanical vibrations to the user's inner ears through bone structure of the user's head and/or through parts of middle ear of the user or electric signals transferred directly or indirectly to cochlear nerve and/or to auditory cortex of the user.

The hearing device is adapted to be worn in any known way. This may include i) arranging a unit of the hearing device behind the ear with a tube leading air-borne acoustic signals into the ear canal or with a receiver/ loudspeaker arranged close to or in the ear canal such as in a Behind-the-Ear type hearing aid, and/ or ii) arranging the hearing device entirely or partly in the pinna and/ or in the ear canal of the user such as in a In-the-Ear type hearing aid or In-the-Canal/ Completely-in-Canal type hearing aid, or iii) arranging a unit of the hearing device attached to a fixture implanted into the skull bone such as in Bone Anchored Hearing Aid or Cochlear Implant, or iv) arranging a unit of the hearing device as an entirely or partly implanted unit such as in Bone Anchored Hearing Aid or Cochlear Implant.

A "hearing system" refers to a system comprising one or two hearing devices, and a "binaural hearing system" refers to a system comprising two hearing devices where the devices are adapted to cooperatively provide audible signals to both of the user's ears. The hearing system or binaural hearing system may further include auxiliary device(s) that communicates with at least one hearing device, the auxiliary device affecting the operation of the hearing devices and/or benefitting from the functioning of the hearing devices. A wired or wireless communication link between the at least one hearing device and the auxiliary device is established that allows for exchanging information (e.g. control and status signals, possibly audio signals) between the at least one hearing device and the auxiliary device. Such auxiliary devices may include at least one of remote controls, remote microphones, audio gateway devices, mobile phones, public-address systems, car audio systems or music players or a combination thereof. The audio gateway is adapted to receive a multitude of audio signals such as from an entertainment device like a TV or a music player, a telephone apparatus like a mobile telephone or a computer, a PC. The audio gateway is further adapted to select and/or combine an appropriate one of the received audio signals (or combination of signals) for transmission to the at least one hearing device. The remote control is adapted to control functionality and operation of the at least one hearing devices. The function of the remote control may be implemented in a SmartPhone or other electronic device, the SmartPhone/ electronic device possibly running an application that controls functionality of the at least one hearing device.

In general, a hearing device includes i) an input unit such as a microphone for receiving an acoustic signal from a user's surroundings and providing a corresponding input audio signal, and/or ii) a receiving unit for electronically receiving an input audio signal. The hearing device further includes a signal processing unit for processing the input audio signal and an output unit for providing an audible signal to the user in dependence on the processed audio signal.

The input unit may include multiple input microphones, e.g. for providing direction-dependent audio signal processing. Such directional microphone system is adapted to enhance a target acoustic source among a multitude of acoustic sources in the user's environment. In one aspect, the directional system is adapted to detect (such as adaptively detect) from which direction a particular part of the microphone signal originates. This may be achieved by using conventionally known methods. The signal processing unit may include amplifier that is adapted to apply a frequency dependent gain to the input audio signal. The signal processing unit may further be adapted to provide other relevant functionality such as compression, noise reduction, etc. The output unit may include an output transducer such as a loudspeaker/ receiver for providing an air-borne acoustic signal transcutaneously or percutaneously to the skull bone or a vibrator for providing a structure-borne or liquid-borne acoustic signal. In some hearing devices, the output unit may include one or more output electrodes for providing the electric signals such as in a Cochlear Implant.

A "cochlear implant system" represents a particular type of a "hearing system" comprising an external unit, which receives acoustic sound and processes the acoustic sound into a coded audio, and an implantable unit which receives the coded audio signal.

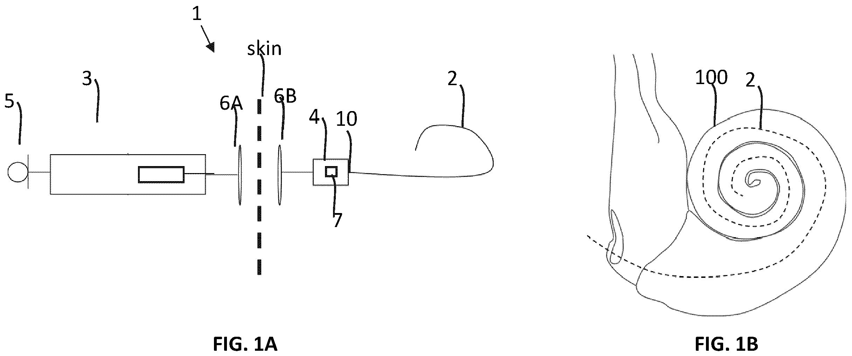

Now referring to FIGS. 1A and 1B illustrating a cochlear hearing aid implant 1 that includes an external part 3, i.e. a sound processor, which includes a microphone 5 and inductive communication link formed by a first inductive coil 6A and a second inductive coil 6B. In this present example the external part 3 is not implanted, however, in another configuration the external part may be fully or partially implanted between the skin and the skull of the patient. Furthermore, the cochlear hearing aid implant 1 includes an implantable part that comprises a housing 4 connected 10 to an electrode lead 2 that comprises a plurality of electrodes. Furthermore, the housing includes an electronic circuit 7 that is configured to provide a stimulation signal based on an inductive signal received by the second transceiver coil 6B. FIG. 1B illustrates an example where the plurality of electrodes is inserted into a cochlea 100 of a patient, and wherein the plurality of electrodes is configured to provide an electrical stimulation of the auditory nerves of a recipient of the cochlear hearing aid implant 1.

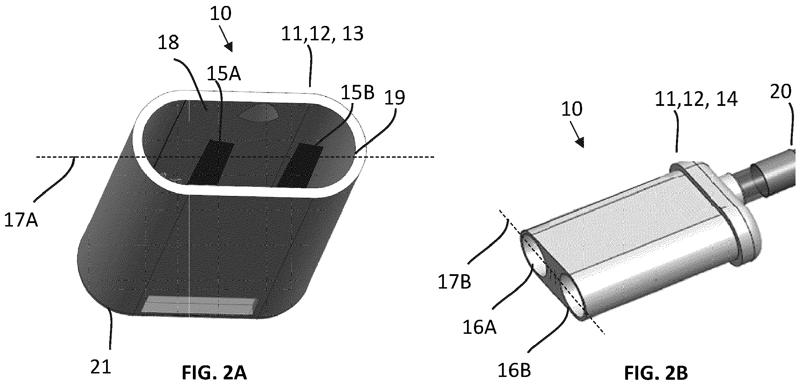

FIGs. 2A and 2B illustrate an example of a connection 10 between the housing 4 and the electrode lead 2provide by a first connector 11 and a second connector 12. FIG. 2A illustrates an example of the first connector 11 or the second connector 12 including a multiple of male plugs 13, and FIG. 2B illustrates an example of the first connector 11 or the second connector 12 including a multiple of female plugs 14. FIG. 2A illustrates an example where the multiple of male plugs 13 includes two male plugs 15A and 15B arranged within a hollow section 18. The hollow section includes an opening 19 configured to receive the multiple female plugs 14 and where the two male plugs (15A, 15B) are configured to be inserted into the female plugs (16A, 16B) of the multiple female plugs 14.

At the opposite end of the opening 19, the multiple male plugs 13 is connected 21 to a conductive path (not illustrated) that is further connected to either the electronic circuit 7 or the electrode lead 2. In another example, the multiple male plugs 13 is connected 21 directly to either the electronic circuit 7 or the electrode lead 2. The female plugs (16A,18B) includes an opening that is configured to receive a male plug (15A, 15B). At the opposite end the opening the female plugs (16A, 16B) are connected 20 to a conductive path (not illustrated) that is further connected to either the electronic circuit 7 or the electrode lead 2. In another example, the multiple female plugs 13 is connected 20 directly to either the electronic circuit 7 or the electrode lead 2.

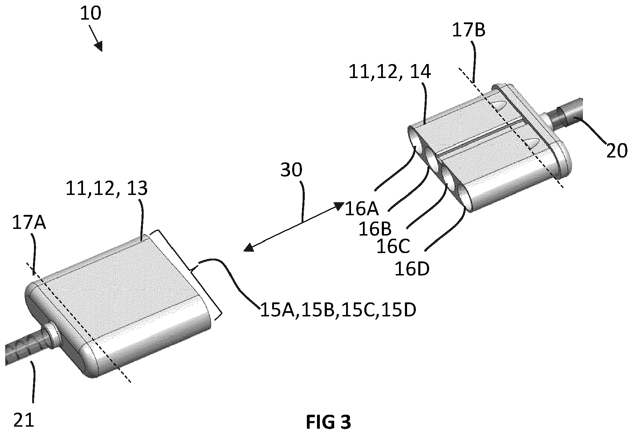

FIG. 3 illustrates an example where the first axis 17A and the second axis 17B are arranged in parallel when being connected 30. In the present example, the multiple male plugs 13 includes four male plugs (15A,15B,15C, 15D), and the multiple female plugs 14 includes four female plugs (16A,16B,16C,16D).

The electrode lead 2 includes a plurality of electrodes that comprises at least 10 electrodes, and wherein the first connector 11 and the second connector 12 are configured to connect the at least 10 electrodes to the electronic circuit 7 when the first connector 11 and the second connector 12 are connected 10.

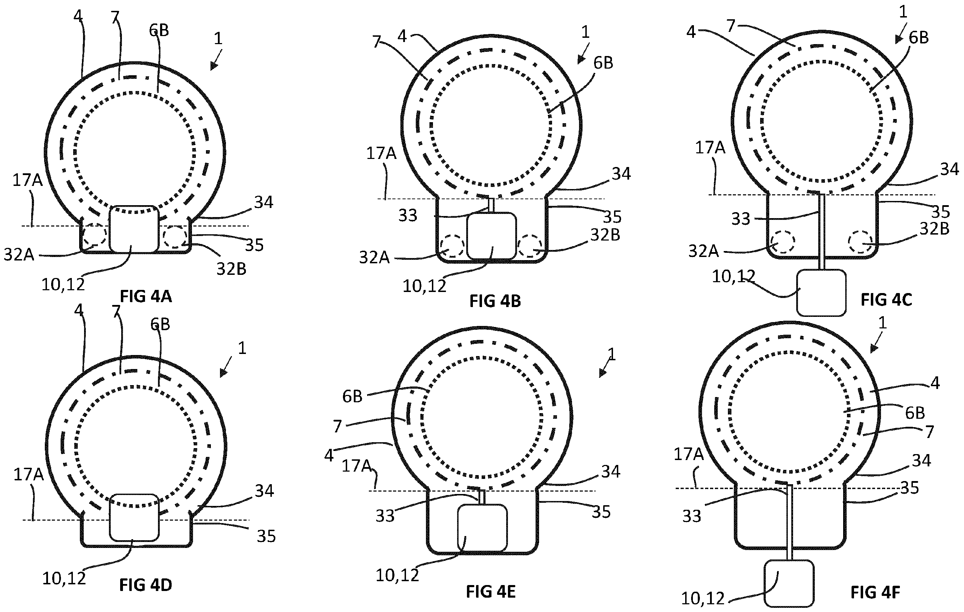

FIGS. 4A to 4F illustrate different examples of a cochlear hearing aid implant 1. The housing 4 includes a part 34 that comprises the electronic circuit 7 and the transceiver coil 6B. Furthermore, the housing may 4 include another part 35 made of a first flexible material that includes at least two fixation means (32A,32B) configured to fixate the housing 4 to the skull via at least two screws. The part 34 that includes the electronic circuit 7 may be covered fully or partially by a second flexible material, where the first flexible material and the second flexible material may be made of the same or different material. The flexible material may be silicone. The thickness of the first flexible material may be thinner than the thickness of the second flexible material for the purpose of making the another part 35 more flexible than the part 34 including the electronic circuit 7. FIG. 4A illustrates an example where the second connector 12 is arranged within the housing 4 fully. The second connector 12 partially overlaps the part 34 and the another part 35. The second connector 12 is arranged between the at least two fixation means (32A,32B), and in FIG. 4D, a similar cochlear hearing aid implant 1 is seen, however, the fixation means (32A,32B) are removed. In the example illustrated in FIG. 4A and 4D, the second connector 12 is arranged either partially between a surface of the housing 4 that is directed towards the skull of the recipient when wearing the implant 1 and the electronic circuit 7. In another example, the second connector 12 is arranged fully between a surface of the housing 4 that is directed towards the skull of the recipient when wearing the implant 1 and the electronic circuit 7.

The second connector 12 does not affect the transceiver coil 6B, and furthermore, the size of the housing 4 is not increased as the second connector 12 utilizes an available free space between the surface of the housing 4 and the electronic circuit 7.

FIG. 4B illustrates an example where the second connector 12 is arranged between the two fixation means and between the electronic circuit 7 and an end of the housing 4 configured to receive 10 the first connector 11. The second connector 12 is then connected to the electronic circuit 7 by a plurality of conductive paths 33 that is configured to establish an electrical connection between the second connector 12 and the electronic circuit 7. FIG. 4E illustrates a similar example as depicted in FIG. 4B, but without the fixation means (32A,32B). In another example, the electronic circuit may be arranged in the another part 35 together with the second connector 12.

FIG. 4C illustrates an example where the second connector 12 is arranged outside the housing 4 and is connected to the electronic circuit 7 via the conductive paths 33. The part of the conductive paths 33 that is outside the housing 4 may be covered by a flexible mean that may be made of a material similar as the housing 4. The conductive paths 33 are arranged between the two fixation means (32A,32B), however, in FIG. 4F the implant 1 does not include the fixation means (32A,32B).

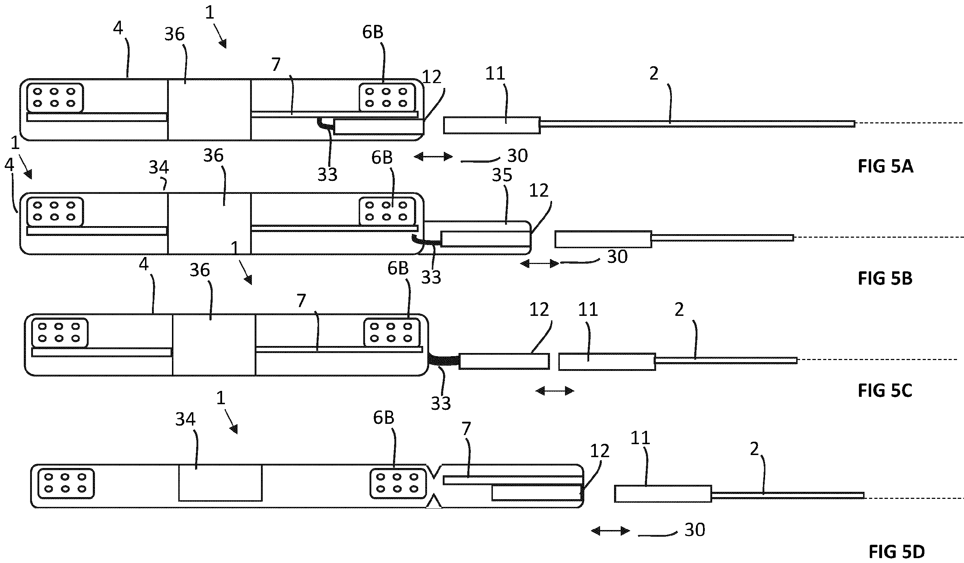

FIGS. 5A to 5D illustrate different examples of the cochlear hearing aid implant 1. FIG. 5A illustrates an example where the second connector 12 is arranged on a first surface of the electronic circuit 7, and on a second surface of the electronic circuit 7 the transceiver coil 6B is arranged. The first surface of the electronic circuit 7 is opposite to the second surface of the electronic circuit 7. The housing 1 includes an insertion 36 for receiving a magnet housing configured to be attract to another magnet arranged in the external part 3. FIG. 5B illustrates an example where the second connector 12 is arranged within the housing 4 and between an opening of the housing 4 configured to receive the first connector 11 and the transceiver coil 6B. The another part 35 of the housing 4 is more flexible than the part 34 of the housing 4 that contains the transceiver coil 6B. FIG. 5C illustrates an example where the second connector 12 is arranged external to the housing 4. In this example, the second connector 4 is connected to the electronic circuit 7 via the conductive paths 33. FIG. 5D illustrates an example where the second connector 12 is arranged on a first surface of the electronic circuit 7, and where the electronic circuit 7 is arranged outside an area circumference by the transceiver coil 6B

In another example, the second connector 12 is connected to the electronic circuit 7 by at least 10 conductive paths 33, and where each of the at least 10 conductive paths 33 is configured to receive the stimulation signal and provide it to the electrode lead 2 when the first connector 11 and the second connector 12 are connected 10.

The at least 10 conductive paths 33 are arranged within a flexible mean (not shown) that is connected to the second connector 12 and to the housing 4.

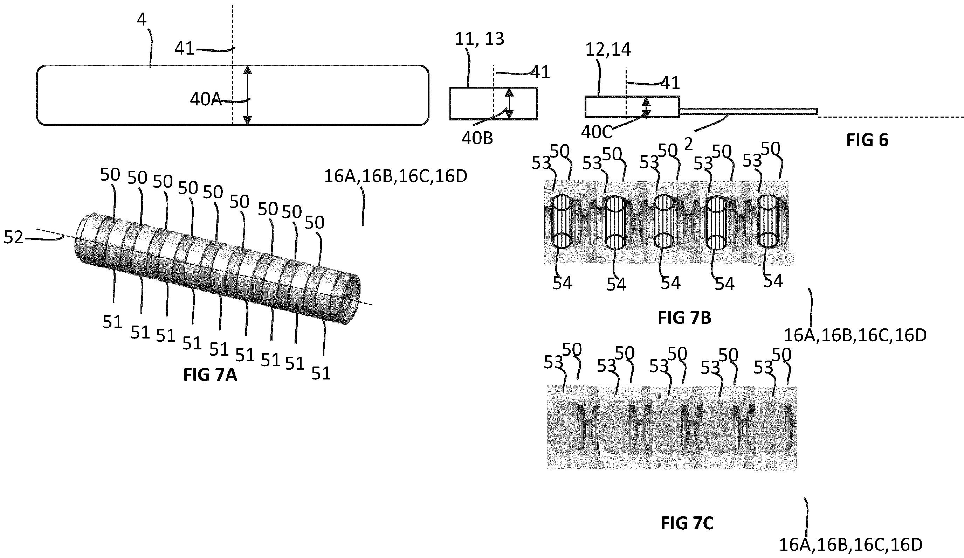

FIG. 6 illustrates an example where the housing 4 has a first thickness 40A, the first connector 11 has a second thickness 40B and the second connector 12 has a third thickness 40C, and wherein the first 40A, second 40B and third 40C thickness are determined along a transverse axis 41, and where the second 40B and third 40C thickness are less than the first thickness 40A. In this example, the first connector 11 includes multiple male plugs 13 and the second connector 12 includes multiple female plugs 14.

FIGS. 7A to 7C illustrates an example of a female plug (16A,16B,16C,16D) of the multiple female plugs 14. The female plug (16A,16B,16C,16D) is a hollow tube that includes multiple electrode female contacts 51 distributed along a longitudinal axis 52 of the hollow tube, and when the hollow tube receives one of the multiple male plugs (15A,15B,15C,15D), each of the multiple electrode female contacts 51 connects to one of the plurality of electrodes via the multiple male plugs (15A,15B,15C,15D). IN this present example, an isolator 50 is arranged between each female contacts 51.

FIG. 7C illustrates an example of a female plug (16A,16B,16C,16D) including multiple female contacts 51, and where each of the female contacts 51 includes a conductive casing 53 with a hollow section configured to deform when receiving one of the multiple male plugs (15A,15B,15C,15D), and wherein the conductive casing 53 is configured to be connected to either the electronic circuit 7 or to an electrode of the plurality of electrodes.

FIG. 7B illustrates an example of a female plug (16A,16B,16C,16D) including multiple female contacts 51, and where each of the female contacts 51 includes a conductive casing 53 configured to be connected to either the electronic circuit 7 or to an electrode of the plurality of electrodes. Furthermore, the female contact 51 includes a flexible member 54 arranged between the conductive casing 53 and an electrode male plug (15A,15B,15C,15D) of the multiple male plugs 13, and wherein the flexible member 54 is configured to deform when receiving one of the multiple male plugs (15A,15B,15C,15D), and wherein the flexible member 54 is made of an electrical conductive material and is electrically connected to the conductive casing 53.

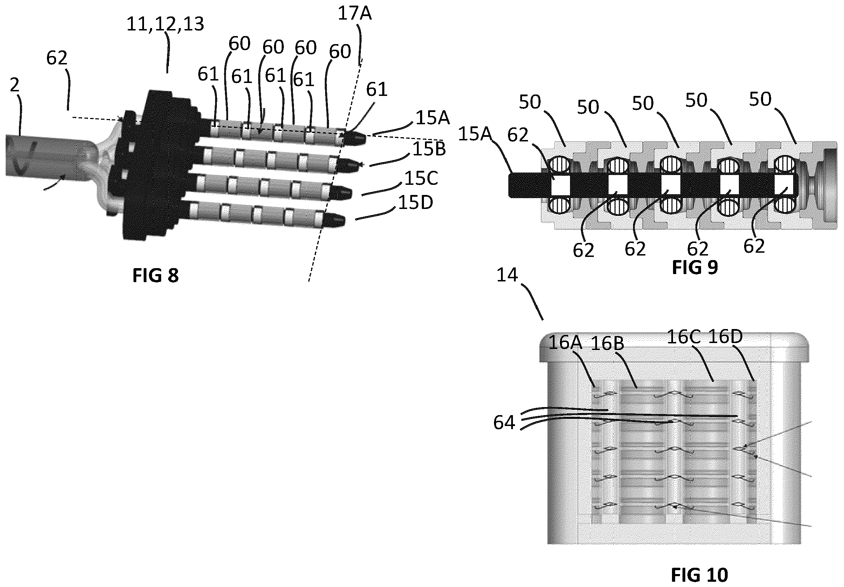

FIG. 8 illustrates an example of multiple electrode male plugs including four electrode male plugs (15A,15B,15C,15D) arranged along a first axis 17A. Each of the electrode male plugs (15A,15B,15C,15D) includes a plurality of electrode male contacts 61 arranged along a longitudinal axis 62 of each of the multiple male plugs (15A,15B,15C,15D), and wherein each of the multiple male plugs (15A,15B,15C,15D) is connected to each of the plurality of electrodes of electrode lead 2. Between each of the electrode male contacts 61 an isolator is arranged for preventing unwanted electrical connection between the male contacts 61.

FIG. 9 illustrates an example where each of the multiple electrode female contacts 50 connects to each of the multiple electrode male contacts 61as the hollow tube of one of the multiple female plugs (16A,16B,16C,16D) receives one of the multiple male plugs (15A, 15B, 15C,15D).

FIG. 10 illustrates an example where each of the multiple female plugs 14 are applied together with a silicon material 64.

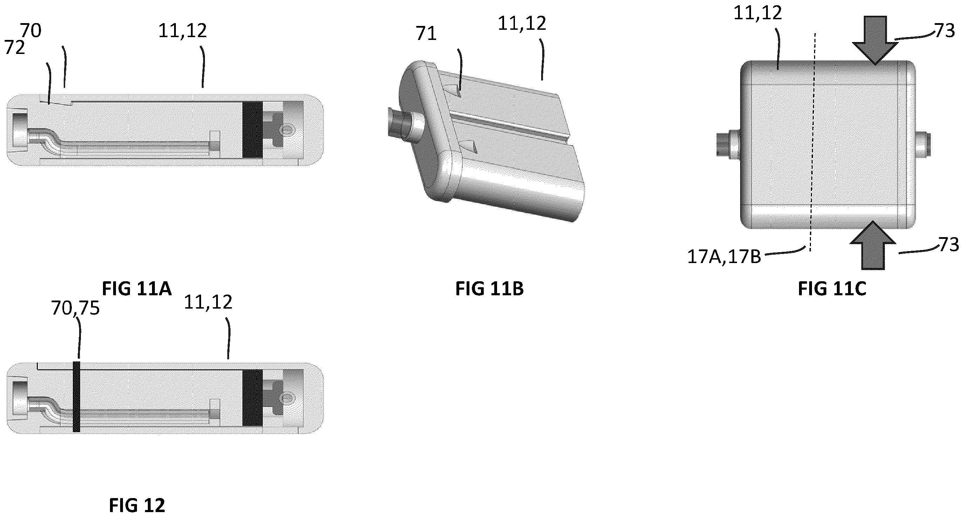

FIGS. 11A to 11C and 12 illustrate different examples of a locking interface 70. The first connector 11 and the second connector 12 includes a locking interface 70 configured to prevent any unwanted disconnections of the first connector 11 and the second connector 12 when being connected 10.

FIGS. 11A to 11C illustrates a locking interface 70 that includes a at least one groove 71 formed into an outer surface of either the first 11 or the second 12 connector and at least one protrusion 72 is arranged on an outer surface of the first 11 or the second 12 connector not having the at least one groove 71, and wherein the at least one grove 71is configured to receive the at least one protrusion 72 when connecting the first 11 and the second 12 connector together.

The at least one protrusion is disengaged from the at least one groove 71 when applying a force 73 onto the outer surface of the first 11 and/or the second 12 connector, and wherein the force 73 is applied particularly parallel to the first 17A and second 17B axis.

FIG. 12 illustrates an example of a locking interface 70, where the locking interface 70 includes a hole formed into the first 11 and the second 12 connector, and wherein the locking interface 70 includes a pin connector 75 configured to be inserted into the hole of the first 11 and the second 12 connector when being connected, and wherein the pin connector 75 is configured to be in a lock position and/or in a disengage position.

A Cochlear hearing aid implant typically includes i) an external part for picking up and processing sound from the environment, and for determining sequences of pulses for stimulation of the electrodes in dependence on the current input sound, ii) a (typically wireless, e.g. inductive) communication link for simultaneously transmitting information about the stimulation sequences and for transferring energy to iii) an implanted part allowing the stimulation to be generated and applied to a number of electrodes, which are implantable in different locations of the cochlea allowing a stimulation of different frequencies of the audible range. Such systems are for example described in US 4,207,441 and in US 4,532,930.

In an aspect, the hearing device comprises multi-electrode array e.g., in the form of a carrier comprising a multitude of electrodes adapted for being located in the cochlea in proximity of an auditory nerve of the user. The carrier is preferably made of a flexible material to allow proper positioning of the electrodes in the cochlea such that the electrodes may be inserted in cochlea of a recipient. Preferably, the individual electrodes are spatially distributed along the length of the carrier to provide a corresponding spatial distribution along the cochlear nerve in cochlea when the carrier is inserted in cochlea.

In still a further aspect, the functions may be stored on or encoded as one or more instructions or code on a tangible computer-readable medium. The computer readable medium includes computer storage media adapted to store a computer program comprising program codes, which when run on a processing system causes the data processing system to perform at least some (such as a majority or all) of the steps of the method described above, in the and in the claims.

As already outlined above, the above described method, including all corresponding exemplary embodiments, for a cochlear implant system may be implemented in softeware.

It is intended that the structural features of the devices described above, either in the detailed description and/or in the claims, may be combined with steps of the method, when appropriately substituted by a corresponding process.

As used, the singular forms "a," "an," and "the" are intended to include the plural forms as well (i.e. to have the meaning "at least one"), unless expressly stated otherwise. It will be further understood that the terms "includes," "comprises," "including," and/or "comprising," when used in this specification, specify the presence of stated features, integers, steps, operations, elements, and/or components, but do not preclude the presence or addition of one or more other features, integers, steps, operations, elements, components, and/or groups thereof. It will also be understood that when an element is referred to as being "connected" or "coupled" to another element, it can be directly connected or coupled to the other element but an intervening elements may also be present, unless expressly stated otherwise. Furthermore, "connected" or "coupled" as used herein may include wirelessly connected or coupled. As used herein, the term "and/or" includes any and all combinations of one or more of the associated listed items. The steps of any disclosed method is not limited to the exact order stated herein, unless expressly stated otherwise.

It should be appreciated that reference throughout this specification to "one embodiment" or "an embodiment" or "an aspect" or features included as "may" means that a particular feature, structure or characteristic described in connection with the embodiment is included in at least one embodiment of the disclosure. Furthermore, the particular features, structures or characteristics may be combined as suitable in one or more embodiments of the disclosure. The previous description is provided to enable any person skilled in the art to practice the various aspects described herein. Various modifications to these aspects will be readily apparent to those skilled in the art, and the generic principles defined herein may be applied to other aspects.

The claims are not intended to be limited to the aspects shown herein, but is to be accorded the full scope consistent with the language of the claims, wherein reference to an element in the singular is not intended to mean "one and only one" unless specifically so stated, but rather "one or more." Unless specifically stated otherwise, the term "some" refers to one or more.

Accordingly, the scope should be judged in terms of the claims that follow.

## Claims

1. A cochlear hearing aid implant configured to stimulate auditory nerves of a recipient of the cochlear hearing aid implant, comprising:
• a transceiver coil configured to at least receive an inductive signal
• an electronic circuit configured to provide a stimulation signal based on the inductive signal,
• an electrode lead including a plurality of electrodes and a first connector, and where the electrode lead is configured to receive the stimulation signal via the first connector and provide the stimulation signal via an electrode of the plurality of electrodes to the auditory nerves,
• a housing configured to accommodate at least the electronic circuit, and wherein the housing includes or is connected to a second connector, and where the first connector and the second connector are configured to be connected, and
wherein the first connector or the second connector includes multiple male plugs arranged in parallel and along a first axis, and wherein the first connector or the second connector includes multiple female plugs arranged in parallel and along a second axis, and where each of the multiple female plugs is configured to be connected to each of the multiple male plug such that the first axis is parallel to the second axis, and where each of the multiple female plugs and each of the multiple male plugs are connected to either the electronic circuit or to an electrode of the plurality of electrodes.

2. A cochlear hearing aid implant according to claim 1, wherein the plurality of electrodes includes at least 10 electrodes, and wherein the first connector and the second connector are configured to connect the at least 10 electrodes to the electronic circuit when the first connector and the second connector are connected.

3. A cochlear hearing aid implant according to any of the previous claims, wherein the second connector is connected to the electronic circuit by at least 10 conductive paths, and where each of the at least 10 conductive paths is configured to receive the stimulation signal and provide it to the electrode lead when the first connector and the second connector are connected.

4. A cochlear hearing aid implant according to claim 3, wherein the at least 10 conductive paths are arranged within a flexible mean that is connected to the second connector and to the housing.

5. A cochlear hearing aid implant according to any of the previous claims, wherein the housing has a first thickness, the first connector has a second thickness and the second connector has a third thickness, and wherein the first, second and third thickness are determined along a transverse axis, and where the second and third thickness are less than the first thickness.

6. A cochlear hearing aid implant according to any of the previous claims, wherein each of the multiple female plugs is a hollow tube that includes multiple electrode female contacts distributed along a longitudinal axis of the hollow tube, and when the hollow tube receives one of the multiple male plugs, each of the multiple electrode female contacts connects to one of the plurality of electrodes.

7. A cochlear hearing aid implant according to claim 6, wherein each of the multiple electrode female contacts includes:
• a conductive casing with a hollow section configured to deform when receiving one of the multiple male plugs, and wherein the conductive casing is configured to be connected to either the electronic circuit or to an electrode of the plurality of electrodes.

8. A cochlear hearing aid implant according to claim 6, wherein each of the multiple electrode female contacts includes:
• a conductive casing configured to be connected to either the electronic circuit or to an electrode of the plurality of electrodes, and
• a flexible member arranged between the conductive casing and an electrode male plug of the multiple male plugs, and wherein the flexible member is configured to deform when receiving one of the multiple male plugs, and wherein the flexible member is made of an electrical conductive material and is electrically connected to the conductive casing.

9. A cochlear hearing aid implant according to any of the previous claims, comprising;
• at least two multiple male plugs arranged in parallel and along the first axis, and
• at least two multiple female plugs arranged in parallel and along the second axis.

10. A cochlear hearing aid implant according to any of the previous claims, wherein each of the multiple male plugs includes multiple electrode male contacts arranged along a longitudinal axis of each of the multiple male plugs, and wherein each of the multiple male plugs is connected to each of the plurality of electrodes.

11. A cochlear hearing aid implant according to claims 6 and 10, wherein each of the multiple electrode female contacts connects to each of the multiple electrode male contacts when the hollow tube receives one of the multiple male plugs.

12. A cochlear hearing aid implant according to claims 6 and 10, comprising isolators arranged between the multiple electrode female contacts and/or the multiple electrode male contacts.

13. A cochlear hearing aid implant according to any of the previous claims, wherein each of the multiple female plugs are applied together with a silicon material.

14. A cochlear hearing aid implant according to any of the previous claims, wherein the first connector and the second connector includes a locking interface configured to prevent any unwanted disconnections of the first connector and the second connector when being connected.

15. A cochlear hearing aid implant according to claim 14, wherein the locking interface includes a at least one groove formed into an outer surface of either the first or the second connector and at least one protrusion is arranged on an outer surface of the first or the second connector not having the at least one groove, and wherein the at least one grove is configured to receive the at least one protrusion when connecting the first and the second connector together.

16. A cochlear hearing aid implant according to claim 15, wherein the at least one protrusion is disengaged from the at least one groove when applying a force onto the outer surface of the first and/or the second connector, and wherein the force is applied particularly parallel to the first and second axis.

17. A cochlear hearing aid implant according to any of the claims 1 to 14, wherein the locking interface includes a hole formed into the first and the second connector, and wherein the locking interface includes a pin connector configured to be inserted into the hole of the first and the second connector when being connected, and wherein the pin connector is configured to be in a lock position and/or in a disengage position.
